# FASCICULE DE BREVET EUROPEEN

(11) **EP 0 975 631 B1**
(45) Date de publication et mention de la délivrance du brevet: **04.10.2001**
(21) Numéro de dépôt: 98920622.2
(22) Date de dépôt: 15.04.1998
(51) Int. Cl.: C07D 451/04, A61K 31/46

(54) **DERIVES DE 5-ARYL-3-(8-AZABICYCLO[3.2.1]OCT-3-YL)-1,3,4-OXADIAZOL-2(3H)-ONE EN TANT QUE LIGANDS DES RECEPTEURS 5-HT4**
5-ARYL-3-(8-AZABICYCLO[3.2.1]OCT-3-YL)-1,3,4-OXADIAZOL-2(3H)-ONE DERIVATE ALS 5-HT4 REZEPTOR LIGANDEN
5-ARYL-3-(8-AZABICYCLO[3.2.1]OCT-3-YL)-1,3,4-OXADIAZOL-2(3H)-ONE DERIVATIVES AS 5-HT4 RECEPTOR LIGANDS

(30) Priorité: 18.04.1997 FR 9704802
(43) Date de publication de la demande: 02.02.2000
(73) Titulaire: SANOFI-SYNTHELABO, 75013 Paris (FR)
(72) Inventeur: JEGHAM, Samir, 34980 Montferricr-sur-Lez (FR); LOCHEAD, Alistair, F-94220 Charenton (FR); GALLI, Frédéric, F-92420 Vaucresson (FR); NEDELEC, Alain, F-92700 Colombes (FR); SAMSON, Axelle, F-75004 Paris (FR); GALLET, Thierry, F-91120 Palaiseau (FR)
(74) Mandataire: Ludwig, Jacques
(86) Numéro de dépôt international: FR9800754
(87) Numéro de publication internationale: WO9847898

(56) Documents cités:
- EP-A- 0 554 794
- EP-A- 0 645 391
- WO-A-93/05038
- WO-A-93/16072
- WO-A-97/17345

## Description

La présente invention a pour objet des composés répondant à la formule générale (I) 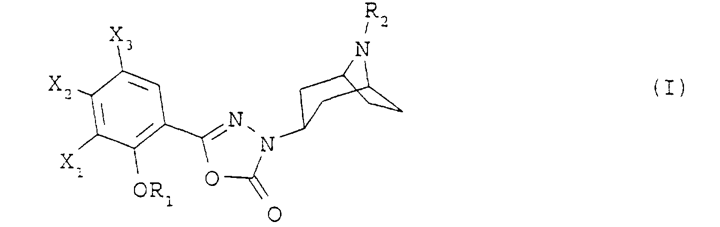 dans laquelle
R₁ représente un groupe (C₁-C₄)alkyle ou (C₃-C₇)cycloalkyl méthyle,
X₁ représente un atome d'hydrogène ou d'halogène ou un groupe (C₁-C₄)alcoxy,
ou bien
OR₁ et X₁ représentent ensemble un groupe de formule -OCH₂O-,
-O(CH₂)₂-, -O(CH₂)₃-, -O(CH₂)₂O- ou -O(CH₂)₃O-,
X₂ représente un atome d'hydrogène ou un groupe amino,
X₃ représente un atome d'hydrogène ou d'halogène, et
R₂ représente soit un atome d'hydrogène, soit un groupe (C₁-C₆)alkyle, soit un groupe phényl (C₁-C₄) alkyle, soit un groupe de formule générale -(CH₂)ₙCO-Z dans laquelle n représente un nombre de 1 à 6 et Z représente un groupe pipéridin-1-yle ou 4-(diméthylamino)pipéridin-1-yle.

Lorsque R₂ représente un groupe alkyle, un tel groupe est, de préférence, un groupe butyle.

Lorsque R₂ représente un groupe phényl(C₁-C₃)alkyle, un tel groupe est, de préférence un groupe 2-phényléthyle.

Lorsque R₂ représente un groupe de formule générale -(CH₂)ₙCO-Z, un tel groupe est, de préférence, un groupe 4-[4-(diméthylamino)pipéridin-1-yl]-4-oxobutyle, un groupe 5-[4-(diméthylamino)pipéridin-1-yl]-5-oxopentyle ou un groupe 6-[4-(diméthylamino)pipéridin-1-yl]-6-oxohexyle.

Les composés de l'invention peuvent exister à l'état de bases libres ou de sels d'addition à des acides pharmaceutiquement acceptables. En raison du cycle tropane, ils peuvent également se présenter sous forme d'isomères *endo* ou *exo.* Par ailleurs, certains substituents R₂ peuvent contenir un atome de carbone asymétrique ; les composés peuvent donc exister sous diverses formes d'isomères géométriques et/ou optiques purs ou en mélanges.

Des *N*-(8-azabicyclo[3.2.1]oct-3-yl)benzamides, de structure relativement proche de celle des composés de l'invention, et ayant des affinités pour les récepteurs 5-HT₃ et 5-HT₄, sont décrits dans la demande de brevet EP-0554794.

Conformément à l'invention on peut préparer les composés de formule générale (I) par un procédé illustré par le schéma qui suit.

On fait réagir un ester de formule générale (II), dans laquelle R₁, X₁, X₂ et X₃ sont tels que définis ci-dessus et R₃ représente un groupe méthyle ou éthyle, avec l'hydrate d'hydrazine, en l'absence de solvant ou dans un solvant polaire protique, par exemple l'éthanol, pour obtenir un hydrazide de formule générale (III) dont on obtient la cyclisation en oxadiazole de formule générale (IV) soit au moyen de phosgène, dans un solvant aprotique, par exemple le dioxane, soit au moyen de chloroformiate de phényle, dans un solvant aprotique, par exemple le toluène.
On fait ensuite réagir l'oxadiazole de formule générale (IV) avec un tropanol de formule générale (V), dans laquelle R₂ est tel que défini à propos de la formule générale (I), mais différent d'un atome d'hydrogène, ou bien représente un groupe protecteur (1,1-diméthyléthoxy)carbonyle, en présence de triphénylphosphine et d'azodicarboxylate d'éthyle, dans un solvant aprotique, par exemple le tétrahydrofurane, puis s'il y a lieu, on déprotège l'azote du cycle tropane au moyen d'acide trifluoroacétique pour obtenir un composé de formule générale (I) dans laquelle R₂ représente un atome d'hydrogène et, si on le désire, on fait réagir le composé obtenu avec un 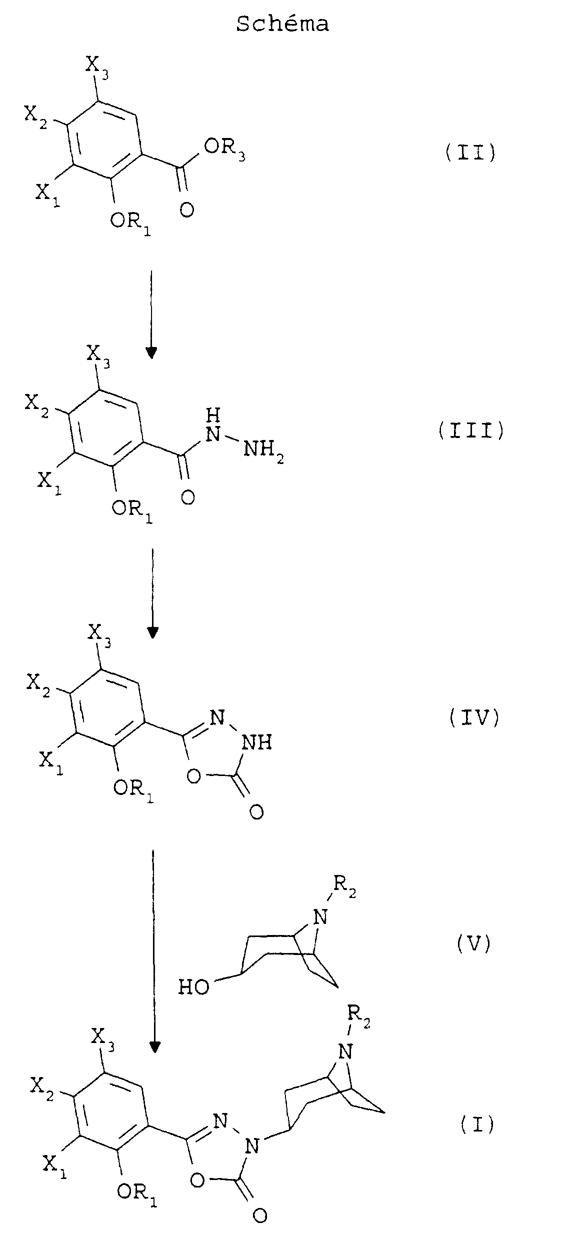 dérivé de formule générale R₂-X, dans laquelle X représente un groupe partant, par exemple un atome d'halogène ou un groupe méthanesulfonate ou paratoluènesulfonate, et R₂ est tel que défini à propos de la formule générale (I), mais différent d'un atome d'hydrogène, en présence de triéthylamine, dans un solvant aprotique, par exemple l'acétonitrile.

Les esters de départ de formule générale (II) et/ou les acides correspondants sont décrits notamment dans les demandes de brevets EP-0231139, EP-0234872, WO-8403281, WO-9316072 et WO-9419344. WO-9316072 décrit également des dérives de benzopyrane qui sont des antagonistes du récepteur 5-HT₄, mais qui ne possèdent ni un groupe oxadiazolone, ni un groupe tropane. Les tropanols de formule générale (V) sont connus ou peuvent être préparés selon toutes méthodes connues. Le 8-[(1,1-diméthyléthoxy)carbonyl]-8-azabicyclo[3.2.1]octan-3-ol peut être préparé par la méthode décrite dans *Drug Metabolism and Disposition* (1992) **20**(4) 596-602.

Les exemples suivants illustrent en détail la préparation de quelques composés selon l'invention. Les microanalyses élémentaires et les spectres IR et RMN confirment les structures des composés obtenus. Les numéros des composés indiqués entre parenthèses dans les titres correspondent à ceux du tableau donné plus loin. Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°1).

5-(4-Amino-5-chloro-2-méthoxyphényl)-3-(8-méthyl-8-aza-bicyclo[3.2.1]oct-3-yl)-1,3,4-oxadiazol-2(3H)-one.

### 1.1. Hydrazide de l'acide 4-amino-5-chloro-2-méthoxyben_ zoïque.

Dans un réacteur de 1 1 on introduit 51,5 g (0,239 mole) de 4-amino-5-chloro-2-méthoxybenzoate de méthyle en suspension dans 460 ml d'éthanol. On ajoute, en 15 min, 119 g (2,39 mole) d'hydrate d'hydrazine et on chauffe le mélange au reflux pendant 15h.
On refroidit le mélange à l'aide d'un bain de glace, on filtre le précipité, on le rince à l'éthanol et on le sèche sous pression réduite à 80°C pendant 2h30.

On obtient ainsi 47,5 g de produit.

Point de fusion : 211°C.

### 1.2. [2-Chloro-5-méthoxy-4-(5-oxo-4,5-dihydro-1,3,4-oxadiazol-2-yl)phényl]carbamate de phénylméthyle.

Dans un réacteur de 3 1 on ajoute, goutte à goutte, en l'espace d'une heure, à température ambiante et sous agitation magnétique, 461 ml (0,875 mole) d'une solution de phosgène 1,93M dans le toluène, à une suspension de 37,7 g (0,175 mole) d'hydrazide de l'acide 4-amino-5-chloro-2-méthoxybenzoïque dans 1200 ml de dioxanne.
On agite le mélange à température ambiante pendant une nuit, puis on le chauffe à 80°C pendant 1h. On chasse l'excès de phosgène par passage d'un courant d'argon à cette température pendant 2h. On ajoute alors 72 ml (0,7 mole) d'alcool benzylique et on continue à chauffer pendant 1h à 100°C. On refroidit, on concentre sous pression réduite et on triture le résidu dans l'éther isopropylique. On filtre et on sèche le solide obtenu. On obtient ainsi 60,3 g de produit.
Point de fusion : 214°C.

### 1.3. Chlorhydrate de [2-chloro-4-[4-(8-méthyl-8-aza-bicyclo[3.2.1]oct-3-yl)-5-oxo-4,5-dihydro-1,3,4-oxadiazol-2-yl] -5-méthoxyphényl]carbamate de phénylméthyle.

Dans un ballon tricol de 50 ml on introduit 1 g (2,66 mmoles) de [2-chloro-5-méthoxy-4-(5-oxo-4,5-dihydro-1,3,4-oxadiazol-2-yl)phényl]carbamate de phénylméthyle, 0,47 g (3,33 mmoles) de endo-8-méthyl-8-azabicyclo[3.2.1]octan-3-ol et 1,05 g (4 mmoles) de triphénylphosphine en solution dans 15 ml de tétrahydrofurane, on refroidit le mélange à 0°C, on ajoute 0,63 ml, soit 0,70 g (4 mmoles) d'azodicarboxylate d'éthyle, et on agite le mélange à 40°C pendant 3 h 30.
On évapore le solvant sous pression réduite, on reprend le résidu avec 15 ml d'un mélange d'éther diéthylique et d'éther diisopropylique, on collecte le solide par filtration. Après rinçage et séchage on obtient 1,6 g de solide dont on prépare le chlorhydrate de manière classique, on triture ce dernier dans l'acétone et on le recristallise dans l'éthanol. On obtient 2,22 g de sel.

### 1.4. 5- (4-Amino-5-chloro-2-méthoxyphényl) -3- (8-méthyl-8-aza-bicyclo[3.2.1]oct-3-yl)-1,3,4-oxadiazol-2(3H)-one.

Dans un ballon tricol contenant 17 ml d'acide acétique on introduit 0,94 g (1,76 mmole) de chlorhydrate de [2-chloro-4-[4-(8-méthyl-8-azabicyclo[3.2.1]oct-3-yl)-5-oxo-4,5-dihydro-1,3,4-oxadiazol-2-yl]-5-méthoxyphényl]carbamate de phénylméthyle, on ajoute 3,12 ml d'acide bromhydrique à 33% dans l'acide acétique (soit 7 équivalents d'acide brom_ hydrique), et on agite le milieu pendant 24 h.
On ajoute 20 ml d'éther diéthylique, on collecte le solide par filtration, on le rince à l'éther diéthylique, on le reprend avec de l'eau, on ajoute de l'ammoniaque jusqu'à pH basique, on obtient un précipité très fin qu'on recueille par filtration. Après séchage on isole 0,45 g de composé.
Point de fusion : 208°C.

### Exemple 2 (Composé N°3).

Chlorhydrate de 5-(8-amino-7-chloro-2,3-dihydro-1,4-benzo dioxin-5-yl)-3-(8-azabicyclo[3.2.1]oct-3-yl)-1,3,4-oxadiazol-2(3*H*)-one.

### 2.1. [6-Chloro-8-[4-[8-[(1,1-diméthyléthoxy)carbonyl)-8-azabicyclo[3.2.1]oct-3-yl]-5-oxo-4,5-dihydro-1,3,4-oxadiazol-2-yl]-2,3-dihydro-1,4-benzodioxin-5-yl)carbamate de phénylméthyle.

On introduit dans un ballon 7,1 g (0,030 mole) de *endo-*8-[(1,1-diméthyléthoxy)carbonyl]-8-azabicyclo[3.2.1]octan-3-ol, 10 g (0,025 mole) de [6-chloro-8-(5-oxo-4,5-dihydro-1,3,4-oxadiazol-2-yl)2,3-dihydro-1,4-benzodioxin-5-yl)carbamate de phénylméthyle (préparé à partir du benzoate de méthyle correspondant selon la méthode décrite dans les étapes 1.1 et 1.2), 8,44 g (0,032 mole) de triphénylphosphine, 5,6 g (0,032 mole) d'azodicarboxylate d'éthyle et 400 ml de tétrahydrofurane sec, et on agite le mélange à température ambiante pendant une nuit. On évapore le solvant sous pression réduite, on purifie le résidu par chromatographie sur gel de silice en éluant avec un mélange 50/50 d'acétate d'éthyle et de heptane et on obtient 13,0 g de composé.
Point de fusion : 210°C.

### 2.2. [6-Chloro-8-[4-(8-azabicyclo[3.2.1]oct-3-yl)-5-oxo-4,5-dihydro-1,3,4-oxadiazol-2-yl]-2,3-dihydro-1,4-benzodioxin-5-yl]carbamate de phénylméthyle.

Dans un ballon tricol de 500 ml on introduit 13,75 g (0,022 mole) de [6-chloro-8-[4-[8-[(1,1-diméthyléthoxy)carbonyl]-8-azabicyclo[3.2.1]oct-3-yl]-5-oxo-4,5-dihydro-1,3,4-oxadiazol-2-yl]-2,3-dihydro-1,4-benzodioxin-5-yl)carbamate de phénylméthyle en solution dans 150 ml de chloroforme, on ajoute lentement, en 20 min, 17,05 ml (0,221 mole) d'acide trifluoroacétique, et on agite le mélange pendant 19 h.
On ajoute encore 17,05 ml (0,221 mole) d'acide trifluoro_ acétique et on agite le mélange pendant 5 h.
On concentre le mélange et on fait cristalliser le résidu dans 300 ml d'éther diéthylique. On reprend le solide avec 100 ml d'eau, on ajoute 3 ml de soude à 30%, on extrait avec du chloroforme et on triture le résidu d'évaporation dans l'éther diisopropylique. On obtient 7,88 g de composé.
Point de fusion : 140°C.

### 2.3. Chlorhydrate de 5-(8-amino-7-chloro-2,3-dihydro-1,4-benzodioxin-5-yl)-3-(8-azabicyclo[3.2.1]oct-3-yl)-1,3,4-oxadiazol-2(3H)-one.

On prépare une suspension de 0,88 g (1,72 mmole) de [6-chloro-8-[4-(8-azabicyclo[3.2.1]oct-3-yl)-5-oxo-4,5-dihydro-1,3,4-oxadiazol-2-yl]-2,3-dihydro-1,4-benzodioxin-5-yl)carbamate de phénylméthyle dans 10 ml d'acide acétique, on ajoute 2,2 ml d'une solution d'acide bromhydrique à 33% dans l'acide acétique, et on agite le tout pendant une nuit.
On ajoute au mélange de l'éther diéthylique, on collecte le précipité par filtration, on le rince à l'éther diéthylique, on le reprend avec de l'eau, on lave la solution avec de l'acétate d'éthyle, on ajoute 0,5 ml de soude aqueuse à 30%, on extrait quatre fois avec du chloroforme, on lave la phase organique à l'eau, on la sèche sur sulfate de sodium, on la filtre et on évapore le solvant. On triture le résidu dans de l'éther diéthylique, on l'essore, on le reprend dans de l'éthanol au reflux, on le traite avec de l'éthanol chlorhydrique. On isole finalement 0,61 g de chlorhydrate.
Point de fusion : 210°C.

### Exemple 3 (Composé N°5) .

Bromhydrate de 5-(8-amino-7-chloro-2,3-dihydro-1,4-benzo_ dioxin-5-yl)-3-(8-butyl-8-azabicyclo[3.2.1]oct-3-yl)-1,3,4-oxadiazol-2(3*H*)-one.

### 3.1. [6-Chloro-8-[4-(8-butyl-8-azabicyclo[3.2.1]oct-3-yl)-5-oxo-4,5-dihydro-1,3,4-oxadiazol-2-yl]-2,3-dihydro-1,4-benzodioxin-5-yl)carbamate de phénylméthyle.

Dans un ballon tricol de 100 ml on introduit 1,54 g (3 mmoles) de [6-chloro-8-(8-azabicyclo[3.2.1]oct-3-yl)-5-oxo-4,5-dihydro-1,3,4-oxadiazol-2-yl]-2,3-dihydro-1,4-benzo_ dioxin-5-yl]carbamate de phénylméthyle et 1,2 g (12 mmoles) de triéthylamine en solution dans 20 ml d'acétonitrile, on ajoute 0,82 g (6 mmoles) de 1-bromobutane et on chauffe le mélange à 60°C pendant 20 h.
On évapore le solvant, on reprend le résidu avec de l'eau, on l'extrait à l'acétate d'éthyle, on lave la phase organique à l'eau, on la sèche, on évapore le solvant sous pression réduite et on purifie le résidu par chromatographie sur gel de silice en éluant avec un mélange 95/5/0,5 de chloroforme, méthanol et ammoniaque. On obtient 1,25 g de composé.
Point de fusion : 142°C.

### 3.2. Bromhydrate de 5-(8-amino-7-chloro-2,3-dihydro-1,4-benzodioxin-5-yl)-3-(8-butyl-8-azabicyclo[3.2.1]oct-3 yl)-1,3,4-oxadiazol-2(3H)-one.

Dans un ballon de 50 ml on introduit 1,2 g (2,11 mmoles) de [6-chloro-8-[4-(8-butyl-8-azabicyclo[3.2.1]oct-3-yl)-5-oxo-4,5-dihydro-1,3,4-oxadiazol-2-yl]-2,3-dihydro-1,4-benzo dioxin-5-yl]carbamate de phénylméthyle, 12 ml d'acide acétique et 3 ml d'acide bromhydrique à 33% dans l'acide acétique et on agite la solution obtenue à température ambiante pendant 18 h.
On ajoute de l'éther diéthylique, on recueille le solide par filtration, en le rinçant à l'éther diéthylique, et on le cristallise dans le propan-2-ol. On obtient 0,92 g de bromhydrate qu'on triture dans l'éthanol, et finalement on isole 0,79 g de composé pur.
Point de fusion : >260°C.

### Exemple 4 (Composé N°4).

5-(8-Amino-7-chloro-2,3-dihydro-1,4-benzodioxin-5-yl)-3-[8-(2-phényléthyl)-8-azabicyclo[3.2.1]oct-3-yl]-1,3,4-oxadiazol-2(*3H*)-one.

### 4.1. [6-Chloro-8-[4-[8-(2-phényléthyl)-8-azabicyclo[3.2.1]_ oct-3-yl]-5-oxo-4,5-dihydro-1,3,4-oxadiazol-2-yl]-2,3-dihydro-1,4-benzodioxin-5-yl)carbamate de phényl_ méthyle.

Dans un ballon tricol de 100 ml on introduit 1,33 g (2,6 mmoles) de [6-chloro-8-[4-(8-azabicyclo[3.2.1]oct-3-yl)-5-oxo-4,5-dihydro-1,3,4-oxadiazol-2-yl]-2,3-dihydro-1,4-benzodioxin-5-yl)carbamate de phénylméthyle, 1,05 g (10,4 mmoles) de triéthylamine et 0,96 g (5,2 mmoles) de (2-bromo_ éthyl)benzène en solution dans 20 ml d'acétonitrile, et on chauffe le mélange à 60°C pendant 18 h.
On ajoute encore 0,48 g (2,6 mmoles) de (2-bromoéthyl)benzène et on continue de chauffer pendant 5 h.
On évapore le solvant sous pression réduite, on reprend le résidu avec de l'eau, on l'extrait au chloroforme, on lave la phase organique à l'eau, on la sèche, on évapore le solvant sous pression réduite et on purifie le résidu par chromatographie sur colonne de gel de silice en éluant avec un mélange 99/1/0,1 de chloroforme, méthanol et ammoniaque.On obtient 1,28 g de produit qui, après cristallisation dans l'éther diisopropylique donne 1,18 g de composé pur.
Point de fusion : 118°C.

### 4.2. 5-(8-Amino-7-chloro-2,3-dihydro-1,4-benzodioxin-5-yl)-3-[8-(2-phényléthyl)-8-azabicyclo[3.2.1]oct-3-yl]-1,3,4-oxadiazol-2(3H)-one.

Dans un ballon de 50 ml on introduit 1,18 g (1,91 mmole) de [6-chloro-8-[4-[8-(2-phényléthyl)-8-azabicyclo[3.2.1]oct-3-yl]-5-oxo-4,5-dihydro-1,3,4-oxadiazol-2-yl]-2,3-dihydro-1,4-benzodioxin-5-yl]carbamate de phénylméthyle, 12 ml d'acide acétique et 2,95 ml d'acide bromhydrique en solution dans de l'acide acétique, et on agite la solution à température ambiante pendant 18 h.
On ajoute de l'éther diéthylique, on recueille le précipité par filtration en le rinçant à l'éther diéthylique, on reprend le bromhydrate brut ainsi obtenu avec 13 ml d'eau et 20 ml de chloroforme, on ajoute 0,5 ml de soude à 30%, on extrait le mélange au chloroforme, on évapore le solvant sous pression réduite et on triture le résidu dans de l'éther diisopropylique. On obtient 0,703 g de composé pur sous forme de base libre.
Point de fusion : 227°C.

### Exemple 5 (Composé N°7).

Chlorhydrate de 3-(8-azabicyclo[3.2.1]oct-3-yl)-5-(6-chloro-3,4-dihydro-2*H*-1-benzopyran-8-yl)-1,3,4-oxadiazol-2(3*H*)-one.

On introduit, dans 100 ml de tétrahydrofurane, 2,83 g (11,2 mmoles) de 5-(6-chloro-3,4-dihydro-2*H*-1-benzopyran-8-yl)-1,3,4-oxadiazol-2(3*H*)-one (préparé à partir du benzoate de méthyle correspondant selon la méthode décrite dans les étapes 1.1 et 1.2), 2,54 g (11,2 mmoles) de *endo*-8-(1,1-diméthyléthoxycarbonyl) -8-azabicyclo [3.2.1]octan-3-ol et 4,11 g (15,68 mmoles) de triphénylphosphine, on refroidit le mélange à 0°C sous atmosphère d'argon, on ajoute 3,0 ml d'azodicarboxylate d'éthyle et on agite le mélange à température ambiante pendant une nuit.
On évapore le solvant sous pression réduite, on reprend le résidu avec 100 ml de dichlorométhane, on ajoute 17,3 ml d'acide trifluoroacétique et on agite le mélange pendant 12 h.
On concentre le mélange à sec, on reprend le résidu avec de l'acide chlorhydrique 1 N, on lave le mélange à l'éther diéthylique puis à l'acétate d'éthyle, on ajoute du carbonate de potassium à la phase aqueuse, et on l'extrait avec du chloroforme. On lave la phase organique, on la sèche, on évapore le solvant sous pression réduite, on reprend le résidu avec 1 équivalent d'éthanol chlorhydrique, on concentre le mélange à sec, on recristallise le résidu dans un mélange 9/1 d'eau et de propan-2-ol. Après filtration et séchage on obtient 1,8 g de composé.
Point de fusion : 254°C.

### Exemple 6 (Composé N°9).

Chlorhydrate de 3-(8-butyl-8-azabicyclo[3.2.1]oct-3-yl)-5-(6-chloro-3,4-dihydro-*2H*-1-benzopyran-8-yl)-1,3,4-oxadiazol-2(3*H*)-one.

On chauffe un mélange de 0,96 g (2,38 mmoles) de chlorhydrate de 3-(8-azabicyclo[3.2.1]oct-3-yl)-5-(6-chloro-3,4-dihydro-2*H*-1-benzopyran-8-yl)-1,3,4-oxadiazol-2(3*H*)-one, 0,28 ml, soit 0,36 g (2,62 mmoles) de bromobutane, 0,72 g (5,24 mmoles) de carbonate de potassium et 40 ml d'acétonitrile à 60°C pendant 24 h.
On évapore le solvant sous pression réduite, on reprend le résidu avec de l'eau, on l'extrait au chloroforme, on lave la phase organique, on la sèche, on évapore le solvant sous pression réduite, on purifie le résidu par chromatographie sur colonne de gel de silice en éluant avec du dichloro_ méthane. On reprend la base avec 1 équivalent d'éthanol chlorhydrique, on concentre la solution, on recristallise le sel dans le propan-2-ol, on l'essore et on le sèche. On obtient 0,38 g de composé.
Point de fusion : 242°C.

### Exemple 7 (Composé N°10).

Chlorhydrate de 3-(8-azabicyclo[3.2.1]oct-3-yl)-5-(5-chloro-2,3-dihydrobenzofuran-7-yl)-1,3,4-oxadiazol-2(3*H*)-one.

On introduit, dans 120 ml de tétrahydrofurane, 3,58 g (15 mmoles) de 5-(5-chloro-2,3-dihydrobenzofuran-7-yl)-1,3,4-oxadiazol-2(3*H*)-one (préparé à partir du benzoate de méthyle correspondant selon la méthode décrite dans les étapes 1.1 et 1.2), 3,41 g (15 mmoles) de *endo*-8-(1,1-diméthyl_ éthoxycarbonyl)-8-azabicyclo[3.2.1]octan-3-ol et 5,51 g (21 mmoles) de triphénylphosphine, on refroidit le mélange à 0°C sous atmosphère d'argon, on ajoute 4,0 ml, soit 4,44 g (25,5 mmoles) d'azodicarboxylate d'éthyle, et on agite le mélange à température ambiante pendant une nuit.
On évapore le solvant sous pression réduite, on reprend le résidu avec 150 ml de dichlorométhane, on ajoute 23,1 ml d'acide trifluoroacétique et on agite pendant 12 h.

On concentre le mélange à sec, on reprend le résidu avec de l'acide chlorhydrique 1 N, on lave le mélange à l'éther diéthylique puis à l'acétate d'éthyle, on ajoute du carbonate de potassium à la phase aqueuse jusqu'à pH=10, et on l'extrait avec du chloroforme. On lave la phase organique, on la sèche, on évapore le solvant sous pression réduite, on reprend le résidu avec 1 équivalent d'éthanol chlorhydrique, on concentre le mélange à sec, on recristallise le résidu dans un mélange 49/1 de propan-2-ol et d'eau. Après filtration et séchage on obtient 1,7 g de composé.
Point de fusion : >260°C.

### Exemple 8 (Composé N°11).

Chlorhydrate de 3-[8-(2-phényléthyl)-8-azabicyclo[3.2.1]oct-3-yl]-5-(5-chloro-2,3-dihydrobenzofuran-7-yl)-1,3,4-oxadiazol-2(3*H*)-one.

On chauffe un mélange de 0,80 g (2,08 mmoles) de chlorhydrate de 3-(8-azabicyclo[3.2.1]oct-3-yl)-5-(5-chloro-2,3-dihydrobenzofuran-7-yl)-1,3,4-oxadiazol-2(3*H*)-one, 0,31 ml, soit 0,43 g (2,29 mmoles) de (2-bromoéthyl)benzène, 0,63 g (4,58 mmoles) de carbonate de potassium et 30 ml d'acéto nitrile à 60°C pendant 24 h.
On évapore le solvant sous pression réduite, on reprend le résidu avec de l'eau, on l'extrait au chloroforme, on lave la phase organique, on la sèche, on évapore le solvant sous pression réduite, on purifie le résidu par chromatographie sur colonne de gel de silice en éluant avec un mélange 98/2 de dichlorométhane et d'éthanol. On reprend la base avec 1 équivalent d'éthanol chlorhydrique, on concentre la solution, on recristallise le sel dans l'éthanol, on l'essore et on le sèche. On obtient 0,84 g de composé.
Point de fusion : 266°C.

### Exemple 9 (Composé N°15).

Bromhydrate de 5-(4-amino-5-chloro-2-méthoxyphényl)-3-(8-azabicycle[3.2.1]oct-3-yl)-1,3,4-oxadiazol-2(3*H*)-one.

### 9.1. [2-Chloro-4-(4-(8-azabicyclo[3.2.1]oct-3-yl)-5-oxo-4,5-dihydro-1,3,4-oxadiazol-2-yl]-5-méthoxyphényl]carbamate de phénylméthyle.

Dans un ballon tricol de 100 ml on introduit 5,78 g (15 mmoles) de [2-chloro-5-méthoxy-4,5-dihydro-1,3,4-oxadiazol-2-yl)phényl]carbamate de phénylméthyle, 6,0 g (23 mmoles) de triphénylphosphine et 3,5 g 15 mmoles) de 8-[(1,1-diméthyl_ éthoxy)carbonyl]-8-azabicyclo[3.2.1]octan-3-ol en solution dans 50 ml de tétrahydrofurane, on refroidit la solution à 0°C et on ajoute, goutte à goutte, 3,64 ml d'azodicarboxylate d'éthyle, on laisse revenir à 20°C et on agite pendant 3 h. On évapore le solvant sous pression réduite, on reprend le résidu dans 50 ml de dichlorométhane, on ajoute, à 0°C, 25 ml d'acide trifluoroacétique et on agite la solution obtenue à température ambiante pendant 4 h.
On évapore le solvant sous pression réduite, on ajoute 50 ml d'eau et 100 ml d'éther diéthylique, on recueille le précipité par filtration, on le rince à l'éther diéthylique et on le sèche.
On obtient 5,0 g de solide blanc.
Point de fusion : 156-157°C.

### 9.2. Bromhydrate de 5-(4-amino-5-chloro-2-méthoxyphényl)-3-(8-azabicyclo[3.2.1]oct-3-yl)-1,3,4-oxadiazol-2(3H)-one.

Dans un ballon de 25 ml on place 5,0 g (10,6 mmoles) de [2-chloro-4-[4-(8-azabicyclo[3.2.1]oct-3-yl)-5-oxo-4,5-dihydro-1,3,4-oxadiazol-2-yl]-5-méthoxyphényl]carbamate de phénylméthyle en solution dans 10 ml d'acide bromhydrique dans l'acide acétique à 33% et on agite à température ambiante pendant24 h.
On ajoute de l'éther diéthylique, on recueille le précipité par filtration et on le rince plusieurs fois à l'éther.
On obtient 4,2 g de composé.
Point de fusion : 235-237°C.

### Exemple 10 (Composé N°13).

(-)-Bistartrate de 5-(4-amino-5-chloro-2-méthoxyphényl)-3-[8-[1- [5-[4-(diméthylamino)pipéridin-1-yl]-5-oxopentyl]-8-azabicyclo[3.2.1]oct-3-yl]-1,3,4-oxadiazol-2(3*H*)-one.

Dans un ballon tricol de 25 ml on introduit 0,5 g (1,16 mmol) de bromhydrate de 5-(4-amino-5-chloro-2-méthoxyphényl)-3-(8-azabicyclo[3.2.1]oct-3-yl)-1,3,4-oxadiazol-2(3*H*)-one, 0,286 g (1,16 mmole) de 1-(5-chloro-1-oxopentyl)-*N*,*N*-diméthylpipéridin-4-amine et 0,484 ml (3,48 mmoles) de triéthylamine en solution dans 10 l de *N,N*-diméthylformamide, et on chauffe le mélange au reflux pendant 18 h.
On évapore le solvant sous pression réduite, on ajoute de l'eau et on extrait au dichlorométhane. On évapore le solvant sous pression réduite et on purifie le résidu par chromatographie sur colonne de gel de silice en éluant avec un mélange 90/10 de dichlorométhane et de méthanol, puis avec un mélange 80/20/2 de dichlorométhane, méthanol et ammoniaque. On obtient une huile qu'on traite avec deux équivalents d'acide tartrique et on isole finalement 0,35 g de solide blanc.
Point de fusion : 198-201°C.

Le tableau qui suit illustre les structures chimiques et les propriétés physiques de quelques composés selon l'invention.

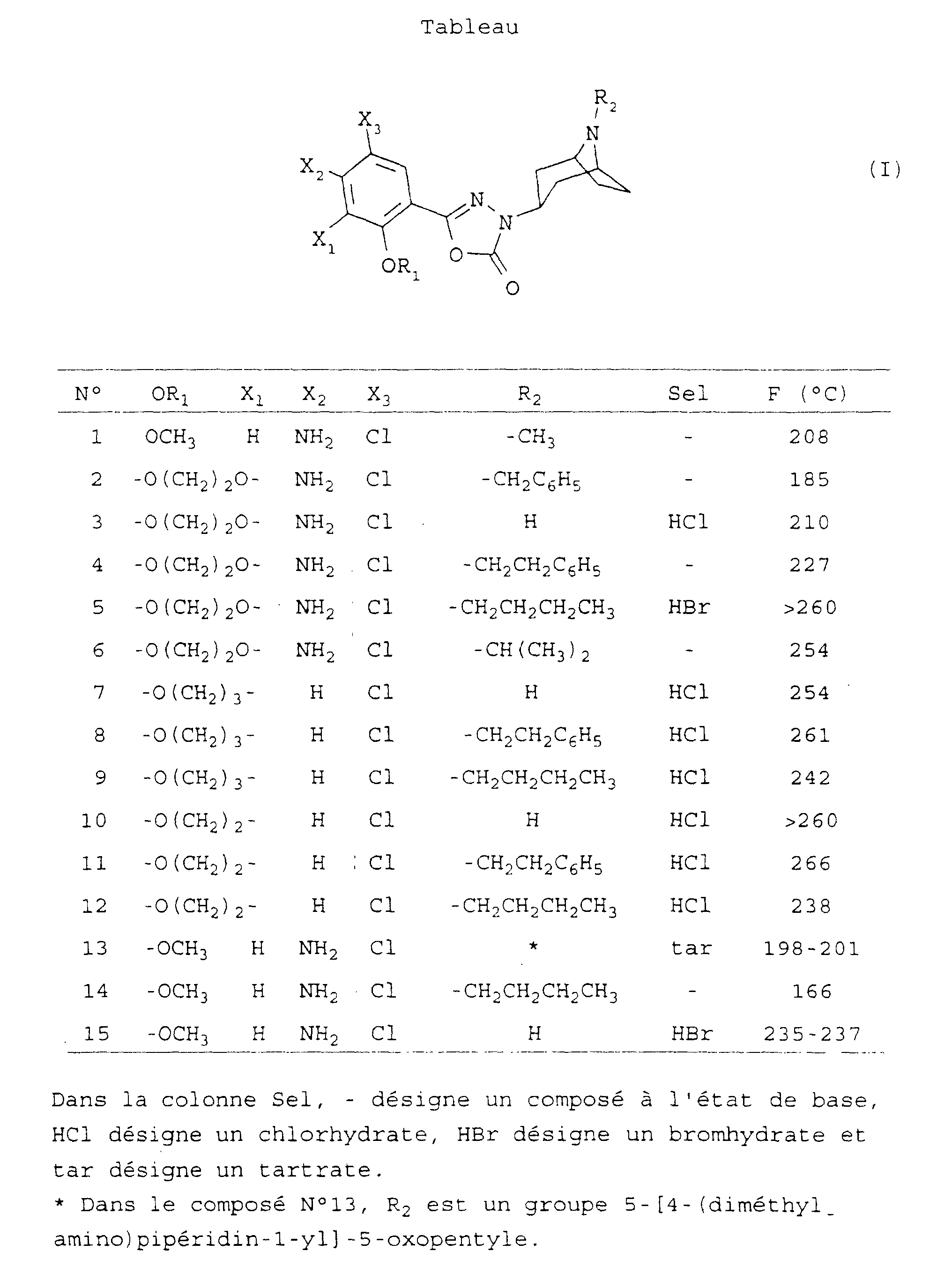

Les composés de l'invention ont fait l'objet d'essais qui ont mis en évidence leur intérêt comme substances à activités thérapeutiques.

Ainsi les composés de l'invention ont été étudiés quant à leur affinité vis-à-vis des récepteurs 5-HT₄ dans le striatum de cobaye selon la méthode décrite par Grossman et coll. dans *Br. J. Pharmacol.* (1993) **109** 618-624.
On euthanasie des cobayes (Hartley, Charles River, France) de 300 à 400 g, on prélève les cerveaux, on excise les striata et on les congèle à -80°C.
Le jour de l'expérience on décongèle le tissu à +4°C dans 33 volumes de tampon HEPES-NaOH (50 mM, pH = 7,4 à 20°C), on l'homogénéise à l'aide d'un broyeur Polytron™, on centrifuge l'homogénat à 48000 g pendant 10 min, on récupère le culot, on le remet en suspension, on le centrifuge de nouveau dans les mêmes conditions et on remet le culot final en suspension dans du tampon HEPES-NaOH, à raison de 30 mg de tissu par ml.
On fait incuber 100 *µ*l de cette suspension membranaire à 0°C pendant 120 min en présence de [³H]GR113808 (ligand décrit dans l'article cité, activité spécifique 80-85 Ci/mmole) dans un volume final de 1 ml de tampon HEPES-NaOH (50 mM, pH = 7,4), en présence ou en absence de composé à tester. On arrête l'incubation par filtration sur filtre Whatman GF/B préalablement traité avec de la polyéthylèneimine à 0,1%, on rince chaque tube avec 4 ml de tampon à 0°C, on filtre de nouveau et on mesure la radioactivité retenue sur le filtre par scintigraphie liquide.
On détermine la liaison non spécifique en présence de sérotonine 30 *µ*M. La liaison spécifique représente 90% de la radioactivité totale récupérée sur le filtre.
Pour chaque concentration de composé étudié on détermine le pourcentage d'inhibition de la liaison spécifique du [³H]GR113808 puis la CI₅₀, concentration du composé testé qui inhibe 50% de la liaison spécifique.
Les CI₅₀ des composés les plus actifs se situent entre 1,3 et 340 nM.

Les composés de l'invention ont aussi été étudiés quant à leurs effets agonistes ou antagonistes vis-à-vis des récepteurs 5-HT₄ dans l'oesophage de rat selon la méthode décrite par Baxter et coll. dans *Naunyn Schmied. Arch. Pharmacol.* (1991) **343** 439.
On utilise des rats mâles Sprague-Dawley pesant de 300 à 450 g. On prélève rapidement un fragment d'environ 1,5 cm de la partie terminale de l'oesophage, on élimine la couche musculaire, on ouvre longitudinalement la tunique muqueuse musculaire interne, on la monte dans une cuve à organe isolé contenant une solution de Krebs-Henseleit à 32°C oxygénée par un courant carbogène (95% O₂ et 5% CO₂), et on la connecte à un transducteur isométrique sous une tension basale de 0,5 g. On induit une contraction du tissu par l'addition de 0,5 *µ*M de carbachol, on attend que la contraction se stabilise (15 min), puis on expose la préparation à la sérotonine (1 *µ*M) afin de quantifier la relaxation maximale. On lave le tissu et, après de 20 min, on ajoute à nouveau 0,5 *µ*M de carbachol, et on expose la préparation au composé à étudier, en concentrations cumulées croissantes de 0,1 à 1 *µ*M.
Les composés qui induisent une relaxation sont considérés comme des agonistes 5-HT₄.
Pour les composés qui n'induisent pas de relaxation, la préparation est exposée à la sérotonine en concentrations cumulées croissantes, de 0,1 nM jusqu'à une concentration induisant une relaxation maximale, et la courbe de relaxation due à la sérotonine, en présence du composé à étudier, est alors comparée à une courbe témoin établie en l'absence dudit composé. Si sa présence induit un déplacement de la courbe vers la droite, le composé étudié est alors considéré comme un antagoniste 5-HT₄.

Les résultats de ces deux essais biologiques montrent que les composés de l'invention sont de puissants ligands des récepteurs sérotoninergiques de type 5-HT₄, et qu'ils agissent sur ces récepteurs soit comme des agonistes, soit comme des antagonistes.

Enfin les composés de l'invention ont fait l'objet d'une étude in vitro quant à leur affinité pour les récepteurs histaminergiques H₃ du cerveau du rat, essentiellement comme décrit par Korte A. et coll., *Biochem. Phys. Res. Commun.* (1990) **168** 979-986, et West R. E. et coll., *Mol. Pharmacol.* (1990) **38** 610-613.

Des rats mâles Sprague Dawley (OFA, Iffa Credo, France), d'un poids de 250 à 300 g, sont euthanasiés et leur cerveau est prélevé. Les tissus sont homogénéisés à l'aide d'un broyeur Polytron™ (position 7, pendnat 20 s) dans 20 volumes de tampon Tris-HCl (50 mM, pH 7,4 à 22°C). L'homogénat est centrifugé à 1000 g pendant 10 min, puis le surnageant est soumis à une nouvelle centrifugation à 45000 g pendant 20 min à 4°C. Le culot est ensuite lavé par remise en suspension dans du tampon, homogénéisation et centrifugation. Le culot final est remis en suspension dans le tampon à raison de 100 mg de tissu initial par millilitre, puis réparti en fractions aliquotes de 11 ml, qui sont congelées à -80°C.
Le jour de l'expérience, la suspension membranaire (100 *µ*l, 300 à 400 *µ*g de protéines) est incubée à 30°C pendant 60 min en présence de 0,5 nM de [³H]N^{α}-méthylhistamine (activité spécifique 75 à 80 Ci/mmole, New England Nuclear, Du Pont de Nemours, Boston, USA) dans un volume final de 500 *µ*l de tampon Tris-HCl, en présence ou en absence de composé à tester. L'incubation est arrêtée par filtration sur filtres Whatman GF/B™ préalablement traités à la polyéthylenimine (0,4%). Chaque tube réactionnel est rincé 3 fois avec 4 ml de tampon Tris-HCl froid (0°C). Les filtres sont séchés dans une étuve à 120°C pendant 5 min. La radioactivité retenue sur les filtres est déterminée par scintigraphie liquide. La liaison non spécifique est déterminée en présence de 10 *µ*M de thioperamide (*N*-cyclohexyl-4-(1*H*-imidazol-4-yl)pipéridine-1-carbothioamide.
Pour chaque concentration de composé étudié, le pourcentage d'inhibition de la liaison spécifique de la [³H]N^{α}-méthyl-histamine est calculé, puis la concentration CI₅₀ de composé inhibant 50% de la liaison est déterminée.

Les composés de l'invention les plus actifs dans cet essai ont une CI₅₀ de l'ordre de 35 nM.

Les résultats des divers essais biologiques effectués sur les composés de l'invention montrent qu'ils sont des ligands des récepteurs 5-HT₄ et/ou des récepteurs H₃.
Ces résultats suggèrent que les composés peuvent être utilisés pour le traitement et la prévention des désordres dans lesquels les récepteurs 5-HT₄ et/ou H₃ sont impliqués, notamment au niveau du système nerveux central, du système
gastro-intestinal, du système du bas appareil urinaire ou du système cardiovasculaire.

Au niveau du système nerveux central, ces désordres et troubles comprennent notamment les troubles neurologiques et psychiatriques tels que les troubles cognitifs, les psychoses, les comportements compulsifs et obsessionnels et les états de dépression et d'anxiété. Les troubles cognitifs comprennent, par exemple, les déficits de mémoire et d'attention, les états de démence (démences séniles du type de la maladie d'Alzheimer ou démences liées à l'âge), les déficiences cérébrales vasculaires, la maladie de Parkinson. Les psychoses comprennent, par exemple, la paranoïa, la schizophrénie, la manie et l'autisme. Les comportements compulsifs et obsessionnels comprennent, par exemple, les troubles alimentaires du type de la boulimie ou de la perte d'appétit. Les états de dépression et d'anxiété comprennent, par exemple, les anxiétés de type anticipatoire (avant intervention chirurgicale, avant traitement dentaire, etc), l'anxiété causée par la dépendance ou le sevrage d'alcool ou de drogue. Enfin on peut citer encore la manie, l'épilepsie, les troubles du sommeil, les désordres affectifs saisonniers, les migraines.

Au niveau du système gastro-intestinal, ces désordres et troubles comprennent notamment les troubles directs ou indirects de la gastromotilité de l'oesophage, de l'estomac ou des intestins, les nausées, les maladies spécifiques comme la dyspepsie, l'ulcère, le reflux gastro-oesophagien, la flatulence, le syndrome du côlon irritable, les troubles de la sécrétion intestinale, les diarrhées, par exemple celles induites par le choléra ou par le syndrome carcinoïde, les désordres liés ou non à la pollution atmosphérique, tels que l'asthme, les rhinites et les difficultés respiratoires.

Au niveau du système du bas appareil urinaire, ces désordres et troubles comprennent notamment les incontinences urinaires, la dysurie, la rétention urinaire.

Au niveau du système cardiovasculaire, ces désordres et troubles comprennent notamment les pathologies liées, directement ou indirectement, aux arythmies cardiaques, à l'hypertension, à l'ischémie, à l'infarctus du myocarde, à l'angine instable, les problèmes de réocclusion après recanalisation, par exemple après thérapie fibrinolytique ou thrombolytique, angioplastie ou chirurgie coronaire.
Le glaucome est également un désordre susceptible d'être traité par les composés de l'invention.

Les composés de l'invention peuvent être présentés sous toutes formes de compositions appropriées à l'administration entérale ou parentérale, telles que comprimés, dragées, gélules, capsules, suspensions ou solutions buvables ou injectables telles que sirops ou ampoules, etc, associés à des excipients convenables, et dosés pour permettre une administration journalière de 0,001 à 20 mg/kg.

## Revendications

1. Composé, sous forme d'isomère géométrique ou optique pur ou de mélange d'isomères, répondant à la formule générale (I) 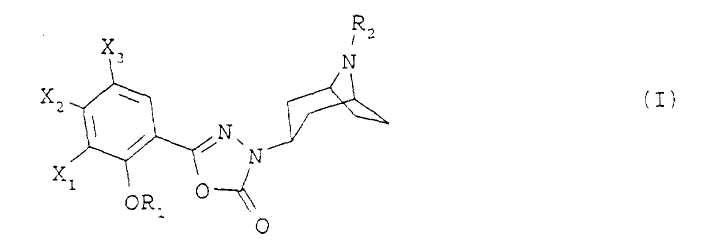 dans laquelle
R₁ représente un groupe (C₁-C₄)alkyle ou (C₃-C₇)cycloalkyl. méthyle,
X₁ représente un atome d'hydrogène ou d'halogène ou un groupe (C₁-C₄) alcoxy,
ou bien
OR₁ et X₁ représentent ensemble un groupe de formule -OCH₂O-,
-O(CH₂)₂-, -O(CH₂)₃-, -O(CH₂)₂O- ou -O(CH₂)₃O-,
X₂ représente un atome d'hydrogène ou un groupe amino,
X₃ représente un atome d'hydrogène ou d'halogène, et
R₂ représente soit un atome d'hydrogène, soit un groupe
(C₁-C₆)alkyle, soit un groupe phényl(C₁-C₄)alkyle, soit un groupe de formule générale -(CH₂)ₙCO-Z dans laquelle n représente un nombre de 1 à 6 et Z représente un groupe pipéridin-1-yle ou 4-(diméthylamino)pipéridin-1-yle, à l'état de base libre ou de sel d'addition à un acide pharmaceutiquement acceptable.

2. Composé selon la revendication 1, **caractérisé en ce que** R₂ représente un groupe butyle.

3. Composé selon la revendication 1, **caractérisé en ce que** R₂ représente un groupe 2-phényléthyle.

4. Composé selon la revendication 1, **caractérisé en ce que** R₂ représente un groupe 4-[4-(diméthylamino)pipéridin-1-yl]-4-oxobutyle, un groupe 5-[4-(diméthylamino)pipéridin-1-yl]-5-oxopentyle ou un groupe 6-[4-(diméthylamino)pipéridin-1-yl]-6-oxohexyle.

5. Procédé de préparation de composés selon la revendication 1, **caractérisé en ce qu'**on fait réagir un ester de formule générale (II) 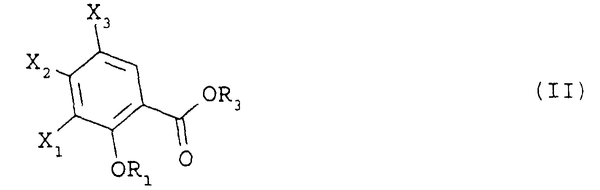 dans laquelle R₁, X₁, X₂ et X₃ sont tels que définis dans la revendication 1 et R₃ représente un groupe méthyle ou éthyle, avec l'hydrate d'hydrazine, pour obtenir un hydrazide de formule générale (III) 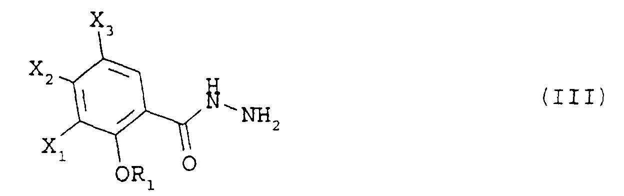 dont on obtient la cyclisation en oxadiazole de formule générale (IV) 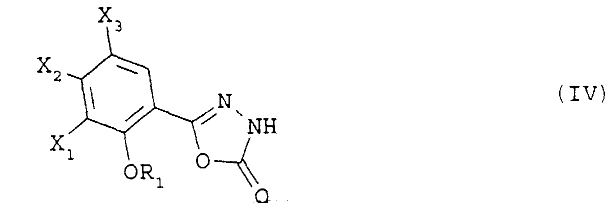 soit au moyen de phosgène, soit au moyen de chloroformiate de phényle, puis on fait réagir l'oxadiazole de formule générale (IV) avec un tropanol de formule générale (V) 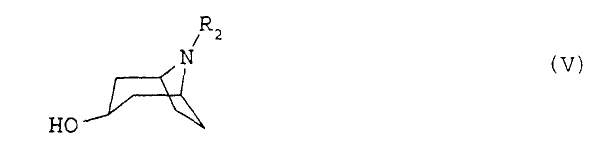 dans laquelle R₂ est tel que défini à propos de la formule générale (I), mais différent d'un atome d'hydrogène, ou bien représente un groupe protecteur (1,1-diméthyléthoxy)-carbonyle, en présence de triphénylphosphine et d'azodicarboxylate d'éthyle, puis s'il y a lieu, on déprotège l'azote du cycle tropane au moyen d'acide trifluoroacétique pour obtenir un composé de formule générale (I) dans laquelle R₂ représente un atome d'hydrogène, et si on le désire, on fait réagir le composé obtenu avec un dérivé de formule générale R₂-X, dans laquelle X représente un groupe partant et R₂ est tel que défini à propos de la formule générale (I), mais différent d'un atome d'hydrogène.

6. Médicament **caractérisé en ce qu'**il est constitué d'un composé selon l'une des revendications 1 à 4.

7. Composition pharmaceutique **caractérisée en ce qu'**elle contient un composé selon l'une des revendications 1 à 4, associé à un excipient.

## Claims

1. Compound, in the form of a pure geometrical or optical isomer or a mixture of isomers, corresponding to the general formula (I) 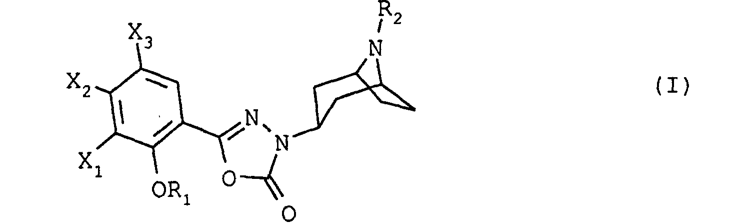 in which
R₁ represents a (C₁-C₄)alkyl or (C₃-C₇)cycloalkylmethyl group,
X₁ represents a hydrogen or halogen atom or a (C₁-C₄)alkoxy group,
or alternatively
OR₁ and X₁ together represent a group of formula -OCH₂O-,
-O(CH₂)₂-, -O(CH₂)₃-, -O(CH₂)₂O- or -O(CH₂)₃O-,
X₂ represents a hydrogen atom or an amino group,
X₃ represents a hydrogen or halogen atom, and
R₂ represents a hydrogen atom, a (C₁-C₄)alkyl group, a phenyl(C₁-C₄)alkyl group, or a group of general formula -(CH₂)ₙCO-Z in which n represents a number from 1 to 6 and Z represents a 1-piperidyl or 4-(dimethylamino)-1-piperidyl group,
in the form of the free base or of an addition salt with a pharmaceutically acceptable acid.

2. Compound according to Claim 1, **characterized in that** R₂ represents a butyl group.

3. Compound according to Claim 1, **characterized in that** R₂ represents a 2-phenylethyl group.

4. Compound according to Claim 1, **characterized in that** R₂ represents a 4-[4-(dimethylamino)-1-piperidyl]-4-oxobutyl group, a 5-[4-(dimethylamino)-1-piperidyl]-5-oxopentyl group or a 6-[4-(dimethylamino)-1-piperidyl]-6-oxohexyl group.

5. Process for the preparation of compounds according to Claim 1, **characterized in that** an ester of general formula (II) 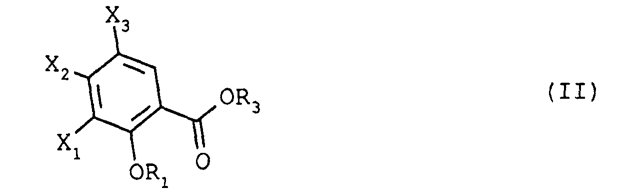 in which R₁, X₁, X₂ and X₃ are as defined in Claim 1 and R₃ represents a methyl or ethyl group, is reacted with hydrazine hydrate, in order to obtain a hydrazide of general formula (III) 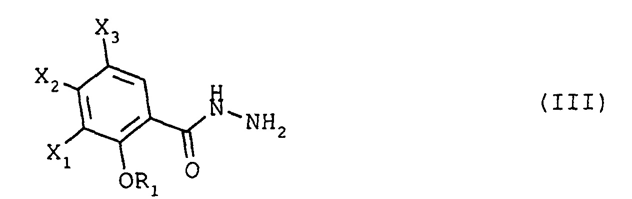 which is cyclized into the oxadiazole of general formula (IV) 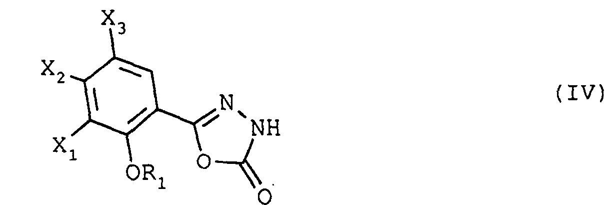 either by means of phosgene or by means of phenyl chloroformate, after which the oxadiazole of general formula (IV) is reacted with with a tropanol of general formula (V) 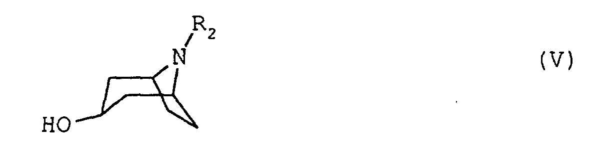 in which R₂ is as defined with respect to the general formula (I), but is other than a hydrogen atom, or alternatively represents a (1,1-dimethylethoxy)carbonyl protecting group, in the presence of triphenylphosphine and ethyl azodicarboxylate, after which, if necessary, the nitrogen of the tropane ring is deprotected by means of trifluoroacetic acid in order to obtain a compound of general formula (I) in which R₂ represents a hydrogen atom, and, if so desired, the compound obtained is reacted with a derivative of general formula R₂-X, in which X represents a leaving group and R₂ is as defined with respect to the general formula (I), but is other than a hydrogen atom.

6. Medicinal product, **characterized in that** it consists of a compound according to one of Claims 1 to 4.

7. Pharmaceutical composition, **characterized in that** it contains a compound according to one of Claims 1 to 4.

## Patentansprüche

1. Verbindung in Form eines reinen geometrischen oder optischen Isomeren oder einer Mischung solcher Isomeren der allgemeinen Formel (I) 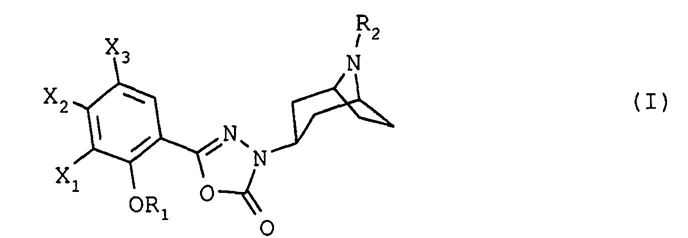 in der
R₁ eine (C₁-C₄)-Alkylgruppe oder eine (C₃-C₇)-Cycloalkylmethylgruppe,
X₁ ein Wasserstoffatom oder Halogenatom, oder eine (C₁-C₄)-Alkoxygruppe, oder OR₁ und X₁ gemeinsam eine Gruppe der Formeln -OCH₂O-, -O(CH₂)₂-, -O(CH₂)₃-, -O(CH₂)₂O- oder -O(CH₂)₃O-,
X₂ ein Wasserstoffatom oder eine Aminogruppe,
X₃ ein Wasserstoffatom oder Halogenatom und
R₂ entweder ein Wasserstoffatom oder eine
(C₁-C₄) -Alkylgruppe, eine Phenyl-(C₁-C₄)-Alkylgruppe oder eine Gruppe der allgemeinen Formel -(CH₂)ₙCO-Z in der n eine Zahl von 1 bis sechs und Z eine Piperidin-1-yl- oder 4-(Dimethylamino)-piperidin-1-yl-gruppe darstellen, bedeuten,
in Form der freien Base oder eines Additionssalzes mit einer pharmazeutisch annehmbaren Säure.

2. Verbindung nach Anspruch 1, **dadurch gekennzeichnet, daß** R₂ eine Butylgruppe bedeutet.

3. Verbindung nach Anspruch 1, **dadurch gekennzeichnet, daß** R₂ eine 2-Phenylethylgruppe bedeutet.

4. Verbindung nach Anspruch 1, **dadurch gekennzeichnet, daß** R₂ eine 4-[4-(Dimethylamino)piperidin-1-yl]-4-oxobutylgruppe, eine 5-[4-(Dimethylamino)piperidin-1-yl]-5-oxopentylgruppe oder eine 6-[4-(Dimethylamino)piperidin-1-yl]-6-oxohexylgruppe bedeutet.

5. Verfahren zur Herstellung der Verbindugen nach Anspruch 1, **dadurch gekennzeichnet, daß** man einen Ester der allgemeinen Formel (II) 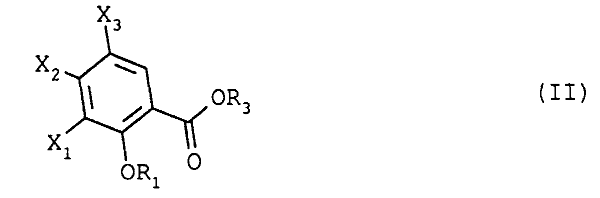 in der R₁, X₁, X₂ und X₃ die in Anspruch 1 angegebenen Bedeutungen besitzen und R₃ eine Methyl- oder Ethylgruppe darstellt, mit Hydrazinhydrat umsetzt zur Bildung eines Hydrazids der allgemeinen Formel (III) 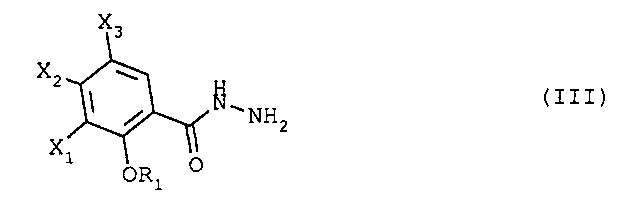 welches man zu einem Oxadiazol der allgemeinen Formel (IV) mit Hilfe entweder von Phosgen oder von Phenylchloroformiat cyclisiert 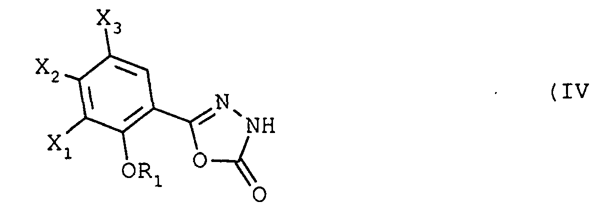 welch letztere Verbindung man mit einem Tropanol der allgemeinen Formel (V) 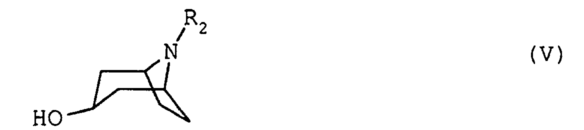 in der R₂ die bezüglich der allgemeinen Formel (I) angegebenen Bedeutungen besitzt mit Ausnahme des Wasserstoffatoms, oder eine (1,1-Dimethylethoxy)-carbonyl-Schutzgruppe darstellt, in Gegenwart von Triphenylphosphin und Azodicarbonsäureethylester umsetzt und erforderlichenfalls die Schutzgruppe am Stickstoffatom des Tropanrings mit Hilfe von Trifluoroessigsäure abspaltet, wobei man eine Verbindung der allgemeinen Formel (I) erhält, in der R₂ ein Wasserstoffatom darstellt, und gewünschtenfalls die erhaltene Verbindung mit einem Derivat der allgemeinen Formel R₂-X, in der X eine austretende Gruppe bedeutet und R₂ die bezüglich der allgemeinen Formel (I) angegebenen Bedeutungen besitzt, mit Ausnahme des Wasserstoffatoms.

6. Arzneimittel, **dadurch gekennzeichnet, daß** es aus einer Verbindung nach einem der Ansprüche 1 bis 4 gebildet ist.

7. Pharmazeutische Zubereitung, **dadurch gekennzeichnet, daß** sie eine Verbindung nach einem der Ansprüche 1 bis 4 zusammen mit einem Trägermaterial enthält.
